# EUROPÄISCHE PATENTSCHRIFT

(11) **EP 1 117 484 B1**
(45) Veröffentlichungstag und Bekanntmachung des Hinweises auf die Patenterteilung: **02.05.2003**
(21) Anmeldenummer: 99944416.9
(22) Anmeldetag: 17.08.1999
(51) Int. Cl.: B01J 37/02, B01J 23/22, C07C 51/265, C07C 51/31

(54) **MEHRSCHICHTIGE SCHALENKATALYSATOREN FÜR DIE GASPHASENOXIDATION VON AROMATISCHEN KOHLENWASSERSTOFFEN**
MULTILAYERED SHELL CATALYSTS FOR CATALYTIC GASEOUS PHASE OXIDATION OF AROMATIC HYDROCARBONS
CATALYSEURS ENROBES MULTICOUCHE POUR L'OXYDATION EN PHASE GAZEUSE D'HYDROCARBURES AROMATIQUES

(30) Priorität: 27.08.1998 DE 19839001
(43) Veröffentlichungstag der Anmeldung: 25.07.2001
(73) Patentinhaber: BASF AKTIENGESELLSCHAFT, 67056 Ludwigshafen (DE)
(72) Erfinder: HEIDEMANN, Thomas, D-69469 Weinheim (DE); BAUER, Stefan, D-67069 Ludwigshafen (DE); LINDEN, Gerd, D-69120 Heidelberg (DE); PETERSEN, Hermann, D-67269 Grünstadt (DE)
(86) Internationale Anmeldenummer: EP9906012
(87) Internationale Veröffentlichungsnummer: WO00012214

(56) Entgegenhaltungen:
- EP-A- 0 021 325
- EP-A- 0 286 448
- DE-A- 2 212 964

## Beschreibung

Die Erfindung betrifft einen Schalenkatalysator für die katalytische Gasphasenoxidation von aromatischen Kohlenwasserstoffen, enthaltend auf einem inerten nicht-porösen Träger eine katalytisch aktive Masse mit einem Gehalt, jeweils bezogen auf die Gesamtmenge der katalytisch aktiven Masse, von 1 bis 40 Gew.-% vanadiumoxid, berechnet als V₂O_{5,} 60 bis 99 Gew.-% Titanoxid, berechnet als TiO₂, bis zu 1 Gew.-% einer Cäsiumverbindung, berechnet als Cs, bis zu 1 Gew.-% einer Phosphorverbindung, berechnet als P und bis zu einem Gesamtgehalt von 10 Gew.-% Antimonoxid, berechnet als Sb₂O₃
Weiterhin betrifft die Erfindung ein Herstellverfahren für diese Katalysatoren und ein Verfahren zur Herstellung von Carbonsauren und/oder Carbonsäureanhydriden und insbesondere von Phthalsäureanhydrid unter Verwendung dieser Katalysatoren.

Bekanntermaßen wird eine Vielzahl von Carbonsäuren und/oder Carbonsäureanhydriden technisch durch die katalytische Gasphasenoxidation von aromatischen Kohlenwasserstoffen, wie Benzol, den Xylolen, Naphthalin, Toluol oder Durol, in Festbettreaktoren, vorzugsweise Rohrbündelreaktoren, hergestellt. Dabei werden beispielsweise Benzoesäure, Maleinsäureanhydrid, Phthalsäureanhydrid, Isophthalsäure, Terephthalsäure oder Pyromellithsäureanhydrid gewonnen. Dazu wird im Allgemeinen ein Gemisch aus einem molekularen Sauerstoff enthaltenden Gas, beispielsweise Luft, und das zu oxidierende Ausgangsmaterial durch eine Vielzahl in einem Reaktor angeordneter Rohre geleitet, in denen sich eine Schüttung mindestens eines Katalysators befindet. Zur Temperaturregelung sind die Rohre von einem Wärmeträgermedium, beispielsweise einer Salzschmelze, umgeben. Trotz dieser Thermostatisierung kann es in der Katalysatorschüttung zur Ausbildung sogenannter "Heißer Flekken" (hot spots) kommen, in denen eine höhere Temperatur herrscht als im übrigen Teil der Katalysatorschüttung. Diese "hot spots" geben Anlaß zu Nebenreaktionen, wie der Totalverbrennung des Ausgangsmaterials oder sie führen zur Bildung unerwünschter, vom Reaktionsprodukt nicht oder nur mit viel Aufwand abtrennbarer Nebenprodukte, beispielsweise zur Bildung von Phthalid oder Benzoesäure, bei der Herstellung von Phthalsäureanhydrid (PSA) aus o-Xylol. Des Weiteren verhindert die Ausbildung eines ausgeprägten hot spots eine schnelles Hochfahren des Reaktors auf die Reaktionstemperatur der umsetzung, da ab einer bestimmten hot spot-Temperatur der Katalysator irreversibel geschädigt werden kann, so dass die Beladungserhöhung nur in kleinen Schritten durchführbar ist und sehr sorgfältig kontrolliert werden muß.

Zur Abschwächung dieses hot spots wurde in der Technik dazu übergegangen, unterschiedlich aktive Katalysatoren schichtweise in der Katalysatorschüttung anzuordnen, wobei in der Regel der weniger aktive Katalysator so im Festbett angeordnet ist, dass das Reaktionsgasgemisch mit ihm als erstes in Kontakt kommt, d.h. er liegt in der Schüttung zum Gaseintritt hin, wohingegen der aktivere Katalysator zum Gasaustritt aus der Katalysatorschüttung hin gelegen ist. Die unterschiedlich aktiven Katalysatoren in der Katalysatorschüttung können bei der gleichen Temperatur dem Reaktionsgas ausgesetzt werden, es können die beiden Schichten aus unterschiedlich aktiven Katalysatoren aber auch auf unterschiedliche Reaktionstemperaturen thermostatisiert mit dem Reaktionsgas in Kontakt gebracht werden (DE-A 40 13 051).

Als Katalysatoren haben sich für diese Oxidationsreaktionen sogenannte Schalenkatalysatoren bewährt, bei denen die katalytisch aktive Masse schalenförmig auf einem im Allgemeinen unter den Reaktionsbedingungen inerten Trägermaterial, wie Quarz (SiO₂), Porzellan, Magnesiumoxid, Zinndioxid, Siliciumcarbid, Rutil, Tonerde (Al₂O₃), Aluminiumsilikat, Magnesiumsilikat (Steatit), Zirkoniumsilikat oder Cersilikat oder Mischungen dieser Trägermaterialien aufgebracht ist. Als katalytisch aktiver Bestandteil der katalytisch aktiven Masse dieser Schalenkatalysatoren dient im Allgemeinen neben Titandioxid in Form seiner Anatasmodifikation Vanadiumpentoxid. Des Weiteren können in der katalytisch aktiven Masse in geringen Mengen eine Vielzahl anderer oxidischer Verbindungen enthalten sein, die als Promotoren die Aktivität und Selektivität des Katalysators beeinflussen, beispielsweise in dem sie seine Aktivität absenken oder erhöhen. Als solche Promotoren seien beispielhaft die Alkalimetalloxide, insbesondere Lithium-, Kalium-, Rubidium- und Cäsiumoxid, Thallium-(I)-oxid, Aluminiumoxid, Zirkoniumoxid, Eisenoxid, Nickeloxid, Kobaltoxid, Manganoxid, Zinnoxid, Silberoxid, Kupferoxid, Chromoxid, Molybdänoxid, Wolframoxid, Iridiumoxid, Tantaloxid, Nioboxid, Arsenoxid, Antimonoxid, Ceroxid und Phosphorpentoxid genannt. Als die Aktivität vermindernder und die Selektivität erhöhender Promotor wirken z.B. die Alkalimetalloxide, wohingegen oxidische Phosphorverbindungen, insbesondere Phosphorpentoxid die Aktivität des Katalysators erhöhen, aber dessen Selektivität vermindern.

Zur Herstellung derartiger Schalenkatalysatoren wird nach den Verfahren von DE-A 16 42 938 und DE-A 17 69 998 eine wäßrige und/oder ein organisches Lösungsmittel enthaltende Lösung oder Suspension der Aktivmassenbestandteile und/oder deren Vorläuferverbindungen, welche im Folgenden als "Maische" bezeichnet wird, auf das Trägermaterial in einer beheizten Dragiertrommel bei erhöhter Temperatur aufgesprüht, bis der gewünschte Aktivmassenanteil am Katalysatorgesamtgewicht erreicht ist. Nach DE 21 06 796 läßt sich die Beschichtung auch in Wirbelbeschichtern durchführen, wie sie z.B. in der DE 12 80 756 angegeben sind. Beim Aufsprühen in der Dragiertrommel sowie beim Beschichten im Wirbelbett treten allerdings hohe Verluste auf, da erhebliche Mengen der Maische vernebelt bzw. durch Abrasion Teile der bereits aufbeschichteten Aktivmasse wieder abgerieben und durch das Abgas ausgetragen werden. Da der Aktivmasseanteil am Gesamtkatalysator im Allgemeinen nur eine geringe Abweichung vom Sollwert haben soll, da durch die Menge der aufgebrachten Aktivmasse und die Schichtdicke der Schale Aktivität und Selektivität des Katalysators stark beeinflußt werden, muß der Katalysator bei den geschilderten Herstellungsweisen häufig zur Bestimmung der aufgebrachten Aktivmassemenge abgekühlt, aus der Dragiertrommel bzw. der Wirbelschicht entnommen und nachgewogen werden. Wird zuviel Aktivmasse auf dem Katalysatorträger abgeschieden, ist im Allgemeinen eine nachträgliche, schonende Entfernung der zu viel aufgetragenen Aktivmassemenge ohne Beeinträchtigung der Festigkeit der Schale, insbesondere ohne Rissbildung in der Katalysatorschale, nicht möglich.

Um diese Probleme abzumildern, wurde in der Technik dazu übergegangen, der Maische organische Binder, bevorzugt Copolymere, vorteilhaft in Form einer wäßrigen Dispersion, von Vinylacetat/Vinyllaurat, Vinylacetat/Acrylat, Styrol/Acrylat, Vinylacetat/Maleat sowie Vinylacetat/Ethylen zuzusetzen, wobei Bindermengen von 10-20 Gew.-%, bezogen auf den Feststoffgehalt der Maische, eingesetzt wurden (EP-A 744 214). Wird die Maische ohne organische Bindemittel auf den Träger aufgetragen, so sind Beschichtungstemperaturen über 150°C von Vorteil. Bei Zusatz oben angegebener Bindemittel liegen die brauchbaren Beschichtungstemperaturen je nach verwendetem Bindemittel zwischen 50 und 450°C (DE 21 06 796). Die aufgetragenen Bindemittel brennen nach dem Einfüllen des Katalysators in den Reaktor und Inbetriebnahme des Reaktors innerhalb kurzer Zeit aus. Der Binderzusatz hat zudem den Vorteil, dass die Aktivmasse gut auf dem Träger haftet, so dass Transport und Einfüllen des Katalysators erleichtert werden.

Gasphasenoxidationen an den oben geschilderten Schalenkatalysatoren finden nicht allein an der äußeren Oberfläche der Schale statt. Zur Erzielung einer für die vollständige Umsetzung der in technischen Verfahren angewandten hohen Beladungen des Reaktionsgases mit Edukt erforderlichen Katalysatoraktivität und -selektivität ist eine effiziente Nutzung der gesamten Aktivmassenschale des Katalysators und damit eine gute Zugänglichkeit des Reaktionsgases zu den in dieser Schale gelegenen Reaktionszentren notwendig. Da die Oxidation aromatischer Verbindungen zu Carbonsäuren und/oder Carbonsäureanhydriden über eine Vielzahl von Zwischenstufen vor sich geht und das Wertprodukt am Katalysator zu Kohlendioxid und Wasser weiteroxidiert werden kann, ist zur Erzielung eines hohen Umsatzes an Edukt unter gleichzeitiger Zurückdrängung des oxidativen Abbaus des Wertprodukts eine optimale Anpassung der Verweilzeit des Reaktionsgases in der Aktivmasse durch die Erzeugung einer geeigneten Aktivmassestruktur (beispielsweise deren Porosität und Porenradienverteilung) in der Katalysatorschale erforderlich.

Des Weiteren ist zu berücksichtigen, dass die Gaszusammensetzung an der äußeren Oberfläche der Aktivmassenschale nicht zwangsläufig auch der Gaszusammensetzung an inneren Stellen der Aktivmasse entspricht. Vielmehr ist zu erwarten-, dass die Konzentration an primären Oxidationsprodukten höher, die Edukt-Konzentration dagegen niedriger ist, als an der äußeren Katalysatoroberfläche. Dieser unterschiedlichen Gaszusammensetzung sollte durch eine gezielte Einstellung der Aktivmassenzusammensetzung innerhalb der Aktivmassenschale Rechnung getragen werden, um zu einer optimalen Katalysatoraktivität und -selektivität zu gelangen. So ist in DE 22 12 964 bereits eine Methode der sequentiellen Aufsprühung von Maischen verschiedener Zusammensetzung und die Verwendung der so erhaltenen Katalysatoren für die Herstellung von Phthalsäureanhydrid beschrieben.

Die so erhaltenen mehrschichtigen Schalenkatalysatoren ergeben aber noch keine befriedigenden Ergebnisse und haben den Nachteil, dass bei ihrer Verwendung zur Oxidation von o-Xylol nur unbefriedigende Ausbeuten an Phthalsäureanhydrid erzielt werden.

Es bestand daher die Aufgabe, mehrschichtige Schalenkatalysatoren herzustellen, die eine weitere Steigerung der Selektivität der Oxidation von aromatischen Kohlenwasserstoffen zu Carbonsäuren erlauben.
Diese Aufgabe wurde gelöst durch einen Schalenkatalysator für die katalytische Gasphasenoxidation von aromatischen Kohlenwasserstoffen, enthaltend auf einem inerten nicht-porösen Träger eine katalytisch aktive Masse mit einem Gehalt, jeweils bezogen auf die Gesamtmenge der katalytisch aktiven Masse, von 1 bis 40 Gew.-% Vanadiumoxid, berechnet als V₂O₅, 60 bis 99 Gew.-% Titanoxid, berechnet als TiO₂, bis zu 1 Gew.-% einer Cäsiumverbindung, berechnet als Cs, bis zu 1 Gew.-% einer Phosphorverbindung, berechnet als P und bis zu einem Gesamtgehalt von 10 Gew.-% Antimonoxid, berechnet als Sb₂O₃, welches dadurch gekennzeichnet ist, dass die katalytisch aktive Masse in zwei oder mehr als zwei Schichten aufgebracht ist, wobei die innere Schicht oder die inneren Schichten einen Antimonoxidgehalt von 1 bis 15 Gew.-% und die äußere Schicht einen demgegenüber um 50 bis 100 % verringerten Antimonoxidgehalt aufweisen und wobei die Menge der katalytisch aktiven Masse der inneren Schicht oder der inneren Schichten 10 bis 90 Gew.-% der Menge der gesamten katalytisch aktiven Masse beträgt.

Weiterhin wurde ein Herstellverfahren für diese Katalysatoren und ein Verfahren zur Herstellung von Carbonsäuren und/oder Carbonsäureanhydriden und insbesondere von Phthalsäureanhydrid unter Verwendung dieser Katalysatoren gefunden.

Die Schichtdicke der inneren Schicht oder die Summe der Schichtdicken der inneren Schichten beträgt in der Regel 0,02 bis 0,2 mm, vorzugsweise 0,05 bis 0,1 mm und der äußeren Schicht 0,02 bis 0,2 mm, vorzugsweise 0,05 bis 0,1 mm.

Die neuen Katalysatoren enthalten bevorzugt zwei konzentrische Schichten aus katalytisch aktiver Masse, wobei die innere Schicht bevorzugt 2 bis 10 und insbesondere 5 bis 10 Gew.-% Vanadiumoxid und bevorzugt 2 bis 7, insbesondere 2,5 bis 5 Gew.-% Antimonoxid und die äußere Schicht bevorzugt 1 bis 5, insbesondere 2 bis 4 Gew.-% Vanadiumoxid und bevorzugt 0 bis 2, insbesondere 0 bis 1 Gew.-% Antimonoxid enthält.

Darüberhinaus enthalten die Schalenkatalysatoren weitere an sich für die Oxidation von aromatischen Kohlenwasserstoffen zu Carbonsäuren bekannte Bestandteile wie Titandioxid in der Anatas-Form mit einer BET-Oberfläche von 5 bis 50 m²/g, vorzugsweise 13 bis 28 m²/g.

Der nicht-poröse Träger aus inertem Material besteht z. B. aus Quarz (SiO₂), Porzellan, Magnesiumoxid, Zinndioxid, Siliciumcarbid, Rutil, Tonerde (Al₂O₃), Aluminiumsilikat, Magnesiumsilikat (Steatit), Zirkoniumsilikat oder Cersilikat oder Mischungen dieser Trägermaterialien. Bevorzugt wird Steatit in Form von Kugeln mit einem Durchmesser von 3 bis 6 mm oder von Ringen mit einem äußeren Durchmesser von 5 bis 9 mm und einer Länge von 4 bis 7 mm verwendet.

Neben den bereits oben genannten fakultativen Zusätzen Cäsium und Phosphor können im Prinzip in der katalytisch aktiven Masse in geringen Mengen eine Vielzahl anderer oxidischer Verbindungen enthalten sein, die als Promotoren die Aktivität und Selektivität des Katalysators beeinflussen, beispielsweise indem sie seine Aktivität absenken oder erhöhen. Als solche Promotoren seien beispielhaft die Alkalimetalloxide, insbesondere außer dem genannten Cäsiumoxid, Lithium-, Kalium- und Rubidiumoxid, Thallium(I)oxid, Aluminiumoxid, Zirkoniumoxid, Eisenoxid, Nickeloxid, Kobaltoxid, Manganoxid, Zinnoxid, Silberoxid, Kupferoxid, Chromoxid, Molybdänoxid, Wolframoxid, Iridiumoxid, Tantaloxid, Nioboxid, Arsenoxid, Antimonoxid, Ceroxid genannt. In der Regel wird jedoch aus dieser Gruppe Cäsium als Promotor verwendet. Ferner kommen von den genannten Promotoren noch bevorzugt als Zusätze die Oxide von Niob, Wolfram und Blei in Mengen von 0,01 bis 0,50 Gew.-%, bezogen auf die katalytisch wirksame Masse, in Betracht. Als die Aktivität erhöhender aber die Selektivität vermindernder Zusatz kommen vor allem oxidische Phosphorverbindungen, insbesondere Phosphorpentoxid, in Betracht.

Dabei ist in der Regel die innere Schicht des Katalysators phosphorhaltig und die äußere Schicht phosphorarm oder phosphorfrei.

Die Aufbringung der einzelnen Schichten des Schalenkatalysators auf den inerten nicht-porösen Träger kann mit beliebigen an sich bekannten Methoden erfolgen, z. B. durch
(a) Aufsprühen von Lösungen oder Suspensionen in der Dragiertrommel,
(b) Beschichtung mit einer Lösung oder Suspension in einer Wirbelschicht oder
(c) Pulverbeschichtung der Träger

### Zu a)

Das sequentielle Aufsprühen erfolgt im Allgemeinen wie in DE 22 12 964 und EP 21325 beschrieben, mit der Maßgabe, dass möglichst Chromatographie-Effekte, d. h. das Auswandern von einzelnen Bestandteilen in die andere Schicht vermieden werden sollte. Falls die aufzubringenden Aktiv-Komponenten nicht zumindest teilweise als unlösliche Metallverbindungen vorliegen, kann es dazu zweckmäßig sein, die aufzubringenden Pulver einer thermischen Vorbehandlung zu unterziehen, bzw. in anderer Weise z.B. durch Zusätze, praktisch unlöslich zu machen.

### Zu b)

Die Beschichtung im Wirbelbett kann wie in DE 12 80 756 beschrieben erfolgen.

### Zu c)

Die Methode der Pulverbeschichtung, die aus WO-A 98/37967 und EP-A 714 700 bekannt ist, kann auch für die sequentielle Beschichtung in mehreren Schichten angewandt werden. Dazu werden aus der Lösung und/oder Suspension der katalytisch aktiven Metalloxide, gegebenenfalls unter Zusatz von Hilfsmitteln, zunächst Pulver hergestellt, die nacheinander, gegebenenfalls mit zwischengeschalteter Wärmebehandlung, schalenförmig auf den Träger aufgebracht werden.

Zur Entfernung flüchtiger Bestandteile wird der Katalysator in der Regel, zumindest anschließend, einer Wärmebehandlung unterzogen.

Die neuen Katalysatoren eignen sich generell zur Gasphasenoxidation aromatischer C₆- bis C₁₀-Kohlenwasserstoffe, wie Benzol, den Xylolen, Toluol, Naphthalin oder Durol (1,2,4,5-Tetramethylbenzol) zu Carbonsäuren und/oder Carbonsäureanhydriden wie Maleinsäureanhydrid, Phthalsäureanhydrid, Benzoesäure und/oder Pyromellithsäuredianhyrid.

Insbesondere ermöglichen die neuen Schalenkatalysatoren eine signifikante Steigerung der Selektivität und Ausbeute bei der Herstellung von Phthalsäureanhydrid.

Zu diesem Zweck werden die erfindungsgemäß hergestellten Katalysatoren in von außen auf die Reaktionstemperatur, beispielsweise mittels Salzschmelzen, thermostatisierte Reaktionsrohre gefüllt und über die so bereitete Katalysatorschüttung das Reaktionsgas bei Temperaturen von im Allgemeinen 300 bis 450, vorzugsweise von 320 bis 420 und besonders bevorzugt von 340 bis 400°C und bei einem Überdruck von im Allgemeinen 0,1 bis 2,5, vorzugsweise von 0,3 bis 1,5 bar mit einer Raumgeschwindigkeit von im Allgemeinen 750 bis 5000 h⁻¹ geleitet.

Das dem Katalysator zugeführte Reaktionsgas wird im Allgemeinen durch Vermischen von einem molekularen Sauerstoff enthaltenden Gas, das außer Sauerstoff noch geeignete Reaktionsmoderatoren und/oder Verdünnungsmittel, wie Dampf, Kohlendioxid und/oder Stickstoff, enthalten kann, mit dem zu oxidierenden, aromatischen Kohlenwasserstoff erzeugt, wobei das den molekularen Sauerstoff enthaltende Gas im Allgemeinen 1 bis 100 , vorzugsweise 2 bis 50 und besonders bevorzugt 10 bis 30 mol-% Sauerstoff, 0 bis 30, vorzugsweise 0 bis 10 mol-% Wasserdampf sowie 0 bis 50, vorzugsweise 0 bis 1 mol-% Kohlendioxid, Rest Stickstoff, enthalten kann. Zur Erzeugung des Reaktionsgases wird das molekularen Sauerstoff enthaltende Gas im Allgemeinen mit 30 g bis 150 g je Nm³ Gas des zu oxidierenden, aromatischen Kohlenwasserstoffs beschickt.

Vorteilhaft wird die Gasphasenoxidation so durchgeführt, dass man zwei oder mehr Zonen, vorzugsweise zwei Zonen der im Reaktionsrohr befindlichen Katalysatorschüttung auf unterschiedliche Reaktionstemperaturen thermostatisiert, wozu beispielsweise Reaktoren mit getrennten Salzbädern, wie sie in DE-A 22 01 528 oder DE-A 28 30 765 beschrieben sind, eingesetzt werden können. Wird die Umsetzung in zwei Reaktionszonen durchgeführt, wie in DE-A 40 13 051 beschrieben, wird im Allgemeinen die zum Gaseintritt des Reaktionsgases hin gelegene Reaktionszone, welche im Allgemeinen 30 bis 80 mol-% des gesamten Katalysatorvolumens umfaßt, auf eine um 1 bis 20, vorzugsweise um 1 bis 10 und insbesondere um 2 bis 8°C höhere Reaktionstemperatur als die zum Gasaustritt hingelegene Reaktionszone thermostatisiert. Alternativ kann die Gasphasenoxidation auch ohne Aufteilung in Temperaturzonen bei einer einzigen Reaktionstemperatur durchgeführt werden. Unabhängig von der Temperaturstrukturierung hat es sich als besonders vorteilhaft erwiesen, wenn in den oben angegebenen Reaktionszonen der Katalysatorschüttung Katalysatoren eingesetzt werden, die sich in ihrer katalytischen Aktivität und/oder chemischen Zusammensetzung ihrer Aktivmasse unterscheiden. Vorzugsweise wird bei Anwendung zweier Reaktionszonen in der ersten, also zum Gaseintritt des Reaktionsgases hin gelegenen Reaktionszone, ein Katalysator eingesetzt, der in Vergleich zum Katalysator, welcher sich in der zweiten, also zum Gasaustritt hin gelegenen Reaktionszone, befindet, eine etwas geringere katalytische Aktivität hat. Im Allgemeinen wird die Umsetzung durch die Temperatureinstellung so gesteuert, dass in der ersten Zone der größte Teil des im Reaktionsgas enthaltenen aromatischen Kohlenwasserstoff bei maximaler Ausbeute umgesetzt wird.

Wird die PSA-Herstellung mit den erfindungsgemäßen Katalysatoren unter Anwendung mehrerer Reaktionszonen, in denen sich unterschiedliche Katalysatoren befinden, durchgeführt, so können in allen Reaktionszonen die neuen Schalenkatalysatoren eingesetzt werden. Es können aber im Allgemeinen bereits erhebliche Vorteile gegenüber herkömmlichen Verfahren erzielt werden, wenn nur in einer der Reaktionszonen der Katalysatorschüttung, beispielsweise der ersten Reaktionszone, ein erfindungsgemäßer Schalenkatalysator verwendet wird und in den übrigen Reaktionszonen, beispielsweise der zweiten oder letzten Reaktionszone, auf herkömmliche Weise hergestellte Schalenkatalysatoren benutzt werden.

### Beispiele

Der verwendete Anatas enthielt: 0,18 Gew.-% S, 0,08 Gew.-% P, 0,24 Gew.-% Nb, 0,01 Gew.-% Na, 0,01 Gew.-% K, 0,004 Gew.-% Zr, 0,004 Gew.-% Pb.

### Beispiel 1: Herstellung des Schalenkatalysators Ia - Vergleich

Aus einer Suspension, bestehend aus 250,0 g Anatas, der eine BET-Oberfläche von 20 m²/g hatte, 13,6 g Vanadyloxalat (= 7,98 g V₂O₅), 1,37 g Cäsiumsulfat (= 1,01 g Cs), 940 g Wasser und 122 g Formamid wurden in einem Sprühtrockner bei einer Gaseintrittstemperatur von 280°C und einer Gasaustrittstemperatur des Trocknungsgases (Luft) von 120°C 270 g Pulver mit einer Partikelgröße von 3 bis 60 µm für 90 Gew.-% des Pulvers hergestellt. Nach Calcination des Pulvers (1 h bei 400°C) wurden 90 g des calcinierten Pulvers mit 10 g Melamin vermischt. 700 g Steatit (Magnesiumsilikat)-Ringe mit einem äußeren Durchmesser von 8 mm, einer Länge von 6 mm und einer Wandstärke von 1,5 mm wurden in einer Dragiertrommel bei 20°C während 20 Minuten mit 93 g des mit Melamin versetzten Pulvers unter Zusatz von 56 g eines 30 Gew.-% Wasser/70 Gew.-% Glycerin-Gemisches beschichtet. Anschließend wurde der so beschichtete Katalysatorträger bei 25°C getrocknet. Das Gewicht der so aufgetragenen katalytisch aktiven Masse betrug nach Wärmebehandlung bei 400°C für 1/2 h 10,7 Gew.-%, bezogen auf das Gesamtgewicht des fertigen Katalysators. Die aufgebrachte, katalytisch aktive Masse, also die Katalysatorschale, bestand aus 0,40 Gew.-% Cäsium (berechnet als Cs), 3,0 Gew.-% Vanadium (berechnet als V₂O₅) und 96,6 Gew.-% Titandioxid.

### Beispiel 2: Herstellung des Schalenkatalysators Ib - Vergleich

Man verfuhr wie in Beispiel 1 beschrieben, verwendete jedoch eine Suspension, die aus 400,0 g Anatas, der eine BET-Oberfläche von 21 m²/g hatte, 57,6 g Vanadyloxalat (= 33,8 g V₂O₅), 2,75 g Cäsiumsulfat (= 2,02 g Cs), 14,4 g Antimontrioxid, 2,5 g Ammoniumdihydrogenphosphat (= 0,67 g P), 1500 g Wasser und 196 g Formamid bestand. Die aufgebrachte, katalytisch aktive Masse bestand aus 0,15 Gew.-% Phosphor (berechnet als P), 7,5 Gew.-% Vanadium (berechnet als V₂O₅), 3,2 Gew.-% Antimon (berechnet als Sb₂O₃), 0,45 Gew.-% Cäsium (berechnet als Cs) und 89,05 Gew.-% Titandioxid.

### Beispiel 3: Herstellung des Schalenkatalysators Ic - Vergleich

Man verfuhr wie in den Beispielen 1 und 2 beschrieben mit der Abänderung, dass zunächst 46 g des unter 1 beschriebenen Pulvers und anschließend 47 g des unter 2 beschriebenen Pulvers auf den Träger aufgetragen wurden.

### Beispiel 4: Herstellung des Schalenkatalysators Id - Vergleich

700 g Steatit (Magnesiumsilikat) Ringe mit einem äußeren Durchmesser von 8 mm, einer Länge von 6 mm und einer Wandstärke von 1,5 mm wurden in einer Dragiertrommel auf 160°C erhitzt und mit einer Suspension aus 400,0 g Anatas mit einer BET-Oberfläche von 20 m²/g, 57,6 g Vanadyloxalat (= 33,8 g V₂O₅), 14,4 g Antimontrioxid, 2,5 g Ammoniumdihydrogenphosphat (= 0,67 g P), 2,44 g Cäsiumsulfat (= 1,79 g Cs), 618 g Wasser und 128 g Formamid besprüht, bis das Gewicht der aufgetragenen Schicht 10,5 % des Gesamtgewichts des fertigen Katalysators betrug. Die auf diese Weise aufgebrachte katalytisch aktive Masse, also die Katalysatorschale, bestand im Mittel aus 0,15 Gew.-% Phosphor (berechnet als P), 7,5 Gew.-% Vanadium (berechnet als V₂O₅), 3,2 Gew.-% Antimon (berechnet als Sb₂O₃), 0,4 Gew.-% Cäsium (berechnet als Cs) und 89,05 Gew.-% Titandioxid.

### Beispiel 5: Herstellung des Katalysators IIa - erfindungsgemäß

Man verfuhr wie in Beispiel 3 beschrieben mit der Maßgabe, dass zunächst 46 g des in Beispiel 2 beschriebenen Pulvers und anschließend 47 g des im Beispiel 1 beschriebenen Pulvers auf den Träger aufgetragen wurden.

### Beispiel 6: Herstellung des Katalysators IIb - erfindungsgemäß

Man verfuhr wie in Beispiel 5 beschrieben mit der Abänderung, dass das Pulver gemäß Beispiel 2.61,5 g statt 57,6 g Vanadyloxalat enthielt.

### Beispiel 7: Herstellung des Katalysators IIc - erfindungsgemäß

Man verfuhr wie in Beispiel 5 beschrieben mit der Abänderung, dass das Pulver gemäß Beispiel 2 20,02 g statt 14,4 g Antimontrioxid enthielt.

### Beispiel 8: Herstellung des Katalysators IId - erfindungsgemäß

Man verfuhr wie in Beispiel 5 beschrieben mit der Abänderung, dass das Pulver gemäß Beispiel 2 9,0 g statt 14,4 g Antimonoxid enthielt.

### Beispiel 9: Herstellung von Katalysator IIe - erfindungsgemäß

700 g Steatit-(Magnesiumsilikat)-Ringe mit einem äußeren Durchmesser von 8 mm, einer Länge von 6 mm und einer Wandstärke von 1,5 mm wurden in einer Dragiertrommel auf 160°C erhitzt und mit einer Suspension aus 400,0 g Anatas mit einer BET-Oberfläche von 20 m²/g, 57,6 g Vanadyloxalat (= 33,8 g V₂O₅), 14,4 g Antimontrioxid, 2,5 g Ammoniumdihydrogenphosphat (= 0,67 g P), 2.44 g Cäsiumsulfat (= 1,79 g Cs), 618 g Wasser und 128 g Formamid besprüht, bis das Gewicht der aufgetragenen Schicht 5.3 % des Gesamtgewichts des fertigen Katalysators betrug. Anschließend wurden diese vorbeschichteten Ringe mit einer Suspension aus 400,0 g Anatas mit einer BET-Oberfläche von 20 m²/g, 30,7 g Vanadyloxalat, 2,45 g Cäsiumsulfat, 618 g Wasser und 128 g Formamid besprüht, bis das Gewicht der aufgetragenen Schicht 10,6 % des Gesamtgewichts des fertigen Katalysators betrug.

### Beispiel 10: Herstellung von Katalysator III - nicht erfindungsgemäß

700 g Steatit-(Magnesiumsilikat)-Ringe mit einem äußeren Durchmesser von 8 mm, einer Länge von 6 mm und einer Wandstärke von 1,5 mm wurden in einer Dragiertrommel auf 160°C erhitzt und mit einer Suspension aus 400,0 g Anatas mit einer BET-Oberfläche von 20 m²/g, 57,6 g Vanadyloxalat, 14,4 g Antimontrioxid, 2,5 g Ammoniumdihydrogenphosphat, 0,61 g Cäsiumsulfat, 618 g Wasser und 128 g Formamid besprüht, bis das Gewicht der aufgetragenen Schicht 10,5 % des Gesamtgewichts des fertigen Katalysators betrug. Die auf diese Weise aufgebrachte katalytisch aktive Masse, also die Katalysatorschale, bestand aus 0,15 Gew.-% Phosphor (berechnet als P), 7,5 Gew.-% Vanadium (berechnet als V₂O₅),
3,2 Gew.-% Antimon (berechnet als Sb₂O₃), 0,1 Gew.-% Cäsium (berechnet als Cs) und 89,05 Gew.-% Titandioxid.

### Beispiel 11: Herstellung von PSA - erfindungsgemäß und Vergleich

1,30 m des Katalysators III und anschließend jeweils 1,60 m der Katalysatoren Ia-Id bzw. IIa-IIe wurden in ein 3,85 m langes Eisenrohr mit einer lichten Weite von 25,2 mm eingefüllt. Das Eisenrohr war zur Temperaturregelung von einer Salzschmelze umgeben. Durch das Rohr wurden stündlich von oben nach unten 4,0 Nm³-Luft mit Beladungen an 98,5 gew.-%igem o-Xylol von 0 bis etwa 85 g/Nm³-Luft geleitet. Dabei wurden bei 75-85 g Beladung die in folgender Tabelle zusammengefaßten Ergebnisse erhalten (Ausbeute bedeutet das erhaltene PSA in Gewichtsprozent, bezogen auf 100 %-iges o-Xylol).

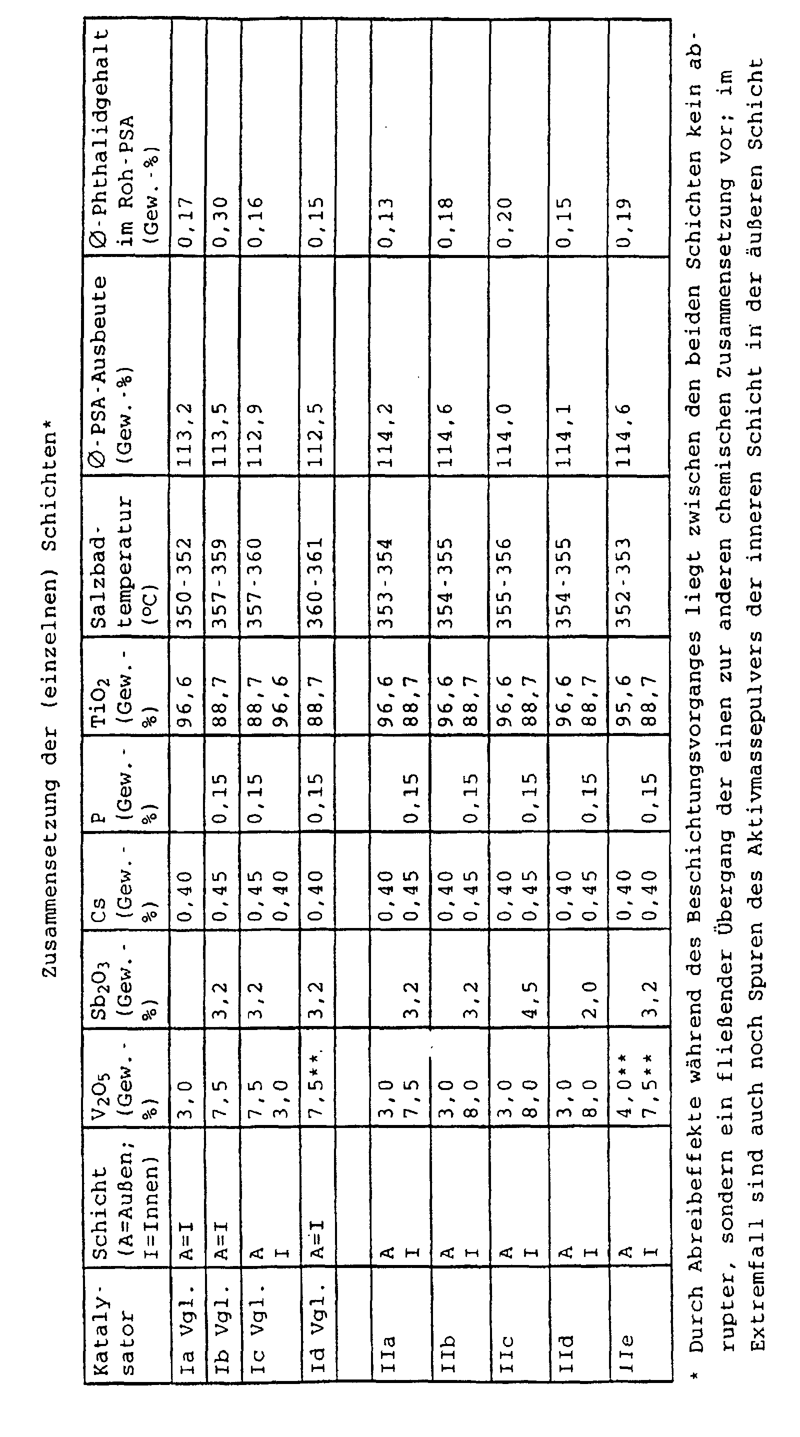

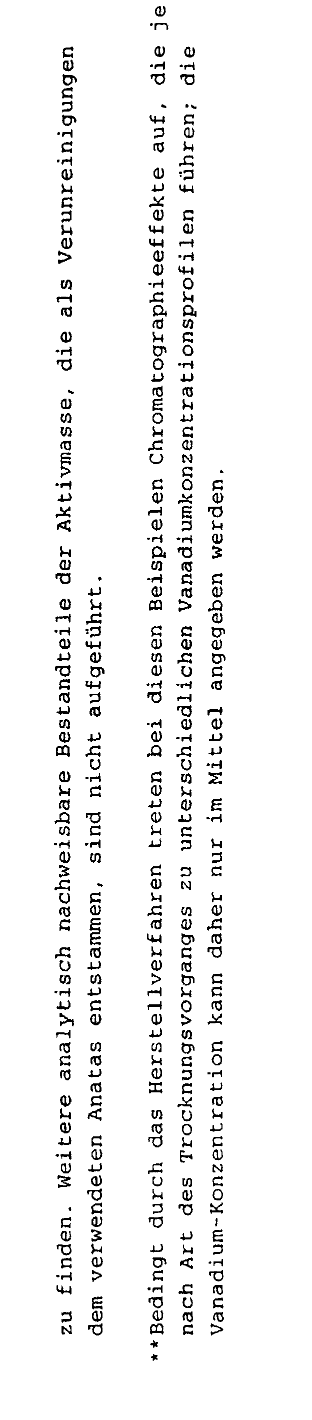

## Patentansprüche

1. Schalenkatalysator für die katalytische Gasphasenoxidation von aromatischen Kohlenwasserstoffen, enthaltend auf einem inerten nicht-porösen Träger eine katalytisch aktive Masse mit einem Gehalt, jeweils bezogen auf die Gesamtmenge der katalytisch aktiven Masse, von 1 bis 40 Gew.-% Vanadiumoxid, berechnet als V₂O₅, 60 bis 99 Gew.-% Titanoxid, berechnet als TiO₂, bis zu 1 Gew.-% einer Cäsiumverbindung, berechnet als Cs, bis zu 1 Gew.-% einer Phosphorverbindung, berechnet als P und einem Gesamtgehalt von größer 0 bis zu 10 Gew.-% Antimonoxid, berechnet als Sb₂O₃, **dadurch gekennzeichnet, dass** die katalytisch aktive Masse in zwei oder mehr als zwei Schichten aufgebracht ist, wobei die innere Schicht oder die inneren Schichten einen Antimonoxidgehalt von 1 bis 15 Gew.-% und die äußere Schicht einen demgegenüber um 50 bis 100 % verringerten Antimonoxidgehalt aufweisen, wobei die Menge der katalytisch aktiven Masse der inneren Schicht oder der inneren Schichten 10 bis 90 Gew.-% der Menge der gesamten katalytisch aktiven Masse beträgt und wobei die Anteile der Bestandteile der katalytisch aktiven Masse aus den angegebenen Bereichen so auszuwählen sind, dass sie sich zu 100 Gew.-% ergänzen.

2. Schalenkatalysator nach Anspruch 1, **dadurch gekennzeichnet, dass** die katalytisch aktive Masse der inneren Schicht oder der Summe der inneren Schichten 30 bis 70 Gew.-% der gesamten katalytisch aktiven Masse des Katalysators ausmacht.

3. Schalenkatalysator nach Anspruch 1, **dadurch gekennzeichnet, dass** die Schichtdicke der inneren Schicht oder die Summe der Schichtdicken der inneren Schichten 0,02 bis 0,2 mm und die Schichtdicke der äußeren Schicht 0,02 bis 0,2 mm beträgt.

4. Schalenkatalysator nach Anspruch 1, **dadurch gekennzeichnet, dass** der Katalysator zwei konzentrische Schichten aus katalytisch aktiver Masse aufweist, wobei die innere Schicht 2 bis 7 Gew.-% Antimonoxid und die äußere Schicht 0 bis 2 Gew.-% Antimonoxid enthält.

5. Schalenkatalysator nach Anspruch 1, **dadurch gekennzeichnet, dass** der Katalysator zwei konzentrische Schichten aus katalytisch aktiver Masse aufweist, wobei die innere Schicht 5 bis 10 Gew.-% Vanadiumoxid und die äußere Schicht 1 bis 5 Gew.-% Vanadiumoxid enthält.

6. Schalenkatalysator nach Anspruch 1, **dadurch gekennzeichnet, dass** das Material des inerten nicht-porösen Trägers Steatit ist.

7. Schalenkatalysator nach Anspruch 1, **dadurch gekennzeichnet, dass** darin das Titanoxid als Titandioxid in der Anatasform mit einer BET-Oberfläche von 13 bis 28 m²/g vorliegt.

8. Schalenkatalysator nach Anspruch 1, **dadurch gekennzeichnet, dass** auf einem inerten nicht-porösen Träger aus Steatit schalenförmig zwei konzentrische Schichten aus katalytisch aktiver Masse aufgebracht sind, wobei neben Titandioxid in der Anatas-Form mit einer BET-Oberfläche von 13 bis 28 m²/g die innere Schicht einen Gehalt von 5 bis 10 Gew.-% Vanadiumoxid, berechnet als V₂O₅, und einen Gehalt von 2 bis 7 Gew.-% Antimonoxid, berechnet als Sb₂O₃, und die äußere Schicht einen Gehalt von 1 bis 5 Gew.-% an Vanadiumoxid, berechnet als V₂O₅, und einen Gehalt von 0 bis 2 Gew.-% Antimonoxid, berechnet als Sb₂O_{3,} aufweist.

9. Verfahren zur Herstellung von Schalenkatalysatoren gemäß Anspruch 1, **dadurch gekennzeichnet, dass** man auf einen inerten nicht-porösen Träger nacheinander zwei oder mehr als zwei Schichten der katalytisch aktiven Masse durch Aufsprühen aufbringt.

10. Verfahren zur Herstellung von Schalenkatalysatoren gemäß Anspruch 1, **dadurch gekennzeichnet, dass** man auf einen inerten nicht-porösen Träger nacheinander zwei oder mehr als zwei Schichten der katalytisch aktiven Masse durch Beschichten mit der einen Binder enthaltenden katalytisch aktiven Masse in Pulverform aufbringt.

11. Verwendung von Katalysatoren gemäß Anspruch 1 zur Herstellung von Carbonsäuren und/oder Carbonsäureanhydriden durch partielle Oxidation von aromatischen Kohlenwasserstoffen.

12. Verfahren zur Herstellung von Phthalsäureanhydrid durch partielle Oxidation von o-Xylol und/oder Naphthalin mit molekularem Sauerstoff enthaltenden Gasen, **dadurch gekennzeichnet, dass** man einen Katalysator gemäß einem der Ansprüche 1 bis 8 verwendet.

## Claims

1. A coated catalyst for the catalytic gas-phase oxidation of aromatic hydrocarbons, comprising, on an inert nonporous support, a catalytically active composition comprising, in each case based on the total amount of catalytically active composition, from 1 to 40% by weight of vanadium oxide, calculated as V₂O₅, from 60 to 99% by weight of titanium dioxide, calculated as TiO₂, up to 1% by weight of a cesium compound, calculated as Cs, up to 1% by weight of a phosphorus compound, calculated as P, and a total of greater than 0 up to 10% by weight of antimony oxide, calculated as Sb₂O₃, wherein the catalytically active composition is applied in two or more layers, where the inner layer or inner layers have an antimony oxide content of from 1 to 15% by weight and the outer layer has, in contrast, an antimony oxide content which is from 50 to 100% lower, where the amount of catalytically active composition of the inner layer or the inner layers is from 10 to 90% by weight of the total amount of catalytically active composition, and where the proportions of constituents in the catalytically active composition are chosen from the given ranges such that they add up to 100% by weight.

2. A coated catalyst as claimed in claim 1, wherein the catalytically active composition of the inner layer or the sum of the inner layers is from 30 to 70% by weight of the total amount of catalytically active composition of the catalyst.

3. A coated catalyst as claimed in claim 1, wherein the thickness of the inner layer or the sum of the thicknesses of the inner layers is from 0.02 to 0.2 mm and the thickness of the outer layer is from 0.02 to 0.2 mm.

4. A coated catalyst as claimed in claim 1, wherein the catalyst has two concentric layers of catalytically active composition, where the inner layer contains from 2 to 7% by weight of antimony oxide and the outer layer contains from 0 to 2% by weight of antimony oxide.

5. A coated catalyst as claimed in claim 1, wherein the catalyst has two concentric layers of catalytically active composition, where the inner layer contains from 5 to 10% by weight of vanadium oxide and the outer layer contains from 1 to 5% by weight of vanadium oxide.

6. A coated catalyst as claimed in claim 1, wherein the material of the inert nonporous support is steatite.

7. A coated catalyst as claimed in claim 1, wherein the titanium oxide therein is present as titanium dioxide in the anatase form and has a BET surface area of from 13 to 28 m²/g.

8. A coated catalyst as claimed in claim 1, wherein two concentric layers of catalytically active composition are applied in the form of a shell to an inert nonporous steatite support, where, apart from titanium dioxide in the anatase form having a BET surface area of from 13 to 28 m²/g, the inner layer comprises from 5 to 10% by weight of vanadium oxide, calculated as V₂O₅, and from 2 to 7% by weight of antimony oxide, calculated as Sb₂O₃, and the outer layer comprises from 1 to 5% by weight of vanadium oxide, calculated as V₂O₅, and from 0 to 2% by weight of antimony oxide, calculated as Sb₂O₃.

9. A process for producing coated catalysts as claimed in claim 1, which comprises applying two or more than two layers of the catalytically active composition in succession to an inert nonporous support by spraying.

10. A process for producing coated catalysts as claimed in claim 1, which comprises applying two or more than two layers of the catalytically active composition in succession to an inert nonporous support by coating with the binder-containing catalytically active composition in powder form.

11. The use of catalysts as claimed in claim 1 for preparing carboxylic acids and/or carboxylic anhydrides by partial oxidation of aromatic hydrocarbons.

12. A process for preparing phthalic anhydride by partial oxidation of o-xylene and/or naphthalene by means of gases comprising molecular oxygen, wherein a catalyst as claimed in any of claims 1 to 8 is used.

## Revendications

1. Catalyseur enrobé pour l'oxydation catalytique en phase gazeuse d'hydrocarbures aromatiques, contenant sur un support inerte non poreux une masse catalytiquement active présentant une teneur, à chaque fois par rapport à la quantité totale de la masse catalytiquement active, de 1 à 40% en poids en oxyde de vanadium, calculée sous forme de V₂O₅, de 60 à 99% en poids en oxyde de titane, calculée sous forme de TiO₂, jusqu'à 1 % en poids en un composé du césium, calculée sous forme de Cs, jusqu'à 1% en poids en un composé du phosphore, calculée sous forme de P et une teneur totale supérieure à 0 jusqu'à 10% en poids en oxyde d'antimoine, calculée sous forme de Sb₂O₃, **caractérisé en ce que** la masse catalytiquement active est appliquée en deux couches ou plus de deux couches, la ou les couches internes présentant une teneur en oxyde d'antimoine de 1 à 15% en poids et la couche extérieure présentant une teneur en oxyde d'antimoine diminuée de 50 à 100% par rapport à celle-ci, la quantité de masse catalytiquement active de la ou des couches internes constituant 10 à 90% en poids de la quantité de masse catalytiquement active totale et les proportions des constituants de la masse catalytiquement active étant choisies dans les plages indiquées de telle manière qu'elles forment 100% en poids.

2. Catalyseur enrobé selon la revendication 1, **caractérisé en ce que** la masse catalytiquement active de la couche interne ou de la somme des couches internes représente 30 à 70% en poids de la masse catalytiquement active totale du catalyseur.

3. Catalyseur enrobé selon la revendication 1, **caractérisé en ce que** l'épaisseur de la couche interne ou la somme des épaisseurs des couches internes est de 0,02 à 0,2 mm et l'épaisseur de la couche externe est de 0,02 à 0,2 mm.

4. Catalyseur enrobé selon la revendication 1, **caractérisé en ce que** le catalyseur présente deux couches concentriques en masse catalytiquement active, la couche interne contenant 2 à 7% en poids d'oxyde d'antimoine et la couche externe 0 à 2% en poids d'oxyde d'antimoine.

5. Catalyseur enrobé selon la revendication 1, **caractérisé en ce que** le catalyseur présente deux couches concentriques en masse catalytiquement active, la couche interne contenant 5 à 10% en poids d'oxyde de vanadium et la couche externe 1 à 5% en poids d'oxyde de vanadium.

6. Catalyseur enrobé selon la revendication 1, **caractérisé en ce que** le matériau du support inerte non poreux est de la stéatite.

7. Catalyseur enrobé selon la revendication 1, **caractérisé en ce que** l'oxyde de titane dans celui-ci se trouve sous forme de dioxyde de titane dans la forme anatase avec une surface BET de 13 à 28 m²/g.

8. Catalyseur enrobé selon la revendication 1, **caractérisé en ce que** deux couches concentriques en masse catalytiquement active sont appliquées en forme de coquille sur un support inerte non poreux en stéatite, la couche interne présentant, outre du dioxyde de titane dans la forme anatase avec une surface BET de 13 à 28 m²/g, une teneur de 5 à 10% en poids en oxyde de vanadium, calculée sous forme de V₂O₅, et une teneur de 2 à 7% en poids en oxyde d'antimoine, calculée sous forme de Sb₂O₃, et la couche externe présentant une teneur de 1 à 5% en poids en oxyde de vanadium, calculée sous forme de V₂O₅ et une teneur de 0 à 2% en poids en oxyde d'antimoine, calculée sous forme de Sb₂O₃.

9. Procédé pour la fabrication de catalyseurs enrobés selon la revendication 1, **caractérisé en ce qu'**on applique sur un support inerte non poreux consécutivement deux couches ou plus de deux couches de la masse catalytiquement active par pulvérisation.

10. Procédé pour la préparation de catalyseurs enrobés selon la revendication 1, **caractérisé en ce qu'**on applique sur un support inerte non poreux consécutivement deux couches ou plus de deux couches de la masse catalytiquement active par revêtement avec une masse catalytiquement active sous forme de poudre contenant un liant.

11. Utilisation de catalyseurs selon la revendication 1 pour la préparation d'acides carboxyliques et/ou d'anhydrides d'acide carboxylique par oxydation partielle d'hydrocarbures aromatiques.

12. Procédé pour la préparation d'anhydride de l'acide phtalique par oxydation partielle d'o-xylène et/ou de naphtalène avec des gaz contenant de l'oxygène moléculaire, **caractérisé en ce qu'**on utilise un catalyseur selon l'une quelconque des revendications 1 à 8.
